# EUROPEAN PATENT APPLICATION

(11) **EP 2 853 586 A1**
(43) Date of publication of application: **01.04.2015**
(21) Application number: 14187016.2
(22) Date of filing: 30.09.2014
(51) Int. Cl.: C12M 1/12, B01D 67/00

(54) **Cell culture carrier and cell culture vessel**

(30) Priority: 30.09.2013 JP 2013205436
(71) Applicant: Fujifilm Corporation, Minato-ku Tokyo 106-8620 (JP)
(72) Inventor: Mizoguchi, Takao, Shizuoka (JP)
(74) Representative: Hoffmann Eitle

(57) **Abstract**

A cell culture carrier comprises an anodic oxide film having a plurality of micropores penetrating the anodic oxide film in a thickness direction, wherein an average density of the plurality of micropores is from 1 micropore/µm² to 15,000 micropores/µm², and an average opening ratio of the anodic oxide film is equal to or higher than 51%.

## Description

### BACKGROUND OF THE INVENTION

This invention relates to a cell culture carrier and a cell culture vessel. Particularly, this invention relates to a cell culture carrier and a cell culture vessel that use an anodic oxide film having a plurality of micropores.

In recent years, as cell culture technologies have advanced, various cell culture carriers for accelerating growth of cells are being developed. A cell culture carrier is generally placed in a culture chamber accommodating a cell culture medium in a cell culture vessel so as to accelerate growth of cells having adhered to the surface of the carrier, thereby maintaining cell viability at a high level.

As such a cell culture carrier, there is a cell culture carrier configured with an anodic oxide film having a plurality of micropores penetrating the film in the thickness direction. For example, JP 2010-226975 A discloses a "membrane for culturing cells (cell culture carrier) having an inorganic material on the surface and/or inside micropores of an anodized oxide film of aluminum" (Claim 1).

As a result of culturing cells by using the cell culture carrier described in JP 2010-226975 A, the inventors of this invention clearly ascertained that the cell viability can be further improved.

### SUMMARY OF THE INVENTION

An object of this invention is to provide a cell culture carrier and a cell culture vessel that can greatly improve cell viability.

In order to achieve the object, the inventors of this invention conducted thorough examination. As a result, they found that when an average opening ratio of the cell culture carrier is controlled to be equal to or higher than 51%, cell viability is greatly improved, and based on this finding, they completed this invention.

That is, this invention provides a cell culture carrier and a cell culture vessel configured as below.
(1) A cell culture carrier comprising an anodic oxide film having a plurality of micropores penetrating the anodic oxide film in a thickness direction, wherein an average density of the plurality of micropores is from 1 micropore/µm² to 15,000 micropores/µm², and an average opening ratio of the anodic oxide film is equal to or higher than 51%.
(2) The cell culture carrier according to (1), wherein an average opening diameter of the plurality of micropores is from 40 nm to 100 nm.
(3) The cell culture carrier according to (1) or (2),
   wherein a thickness of the cell culture carrier is from 10 µm to 300 µm.
(4) A cell culture vessel comprising: at least one culture well having a culture chamber accommodating a cell culture medium; and the cell culture carrier according to any one of (1) to (3) that has a front surface to which cells adhere and which is positioned inside the culture chamber, and that is disposed such that the cell culture medium fills the cell culture carrier from the front surface to a rear surface of the cell culture carrier.
(5) The cell culture vessel according to (4), wherein the cell culture carrier is disposed such that the front surface and the rear surface thereof are positioned inside the culture chamber.
(6) The cell culture vessel according to (4), further comprising an accommodation portion that has an accommodation chamber accommodating the cell culture medium in an amount greater than that of the cell culture medium accommodated in the culture chamber, wherein the cell culture carrier configures at least part of a bottom plate of the culture well, the bottom plate of the culture well is disposed so as to be positioned inside the accommodation chamber, and the culture chamber is in communication with the accommodation chamber through the plurality of micropores.
(7) The cell culture vessel according to (6), wherein the culture well has an expansion portion that is in the form of a flat plate extending toward a side portion of the accommodation portion from a side edge of the bottom plate, and the accommodation portion has a supporter that supports the expansion portion in the side portion thereof.
(8) The cell culture vessel according to (7), wherein the expansion portion is formed of another anodic oxide film having a plurality of micropores penetrating the another anodic oxide film in a thickness direction and is integrally formed with the cell culture carrier.

According to this invention, it is possible to provide a cell culture carrier and a cell culture vessel that can greatly improve cell viability.

### BRIEF DESCRIPTION OF THE DRAWINGS

FIG. 1A is a front view showing an example of a preferable embodiment of the cell culture carrier of this invention, and FIG. 1B is a cross-sectional view taken along line Ib-Ib of FIG. 1A.
FIG. 2 is a cross-sectional view showing a modification example of the cell culture carrier of this invention.
FIG. 3 is a cross-sectional view showing another modification example of the cell culture carrier of this invention.
FIG. 4 is a cross-sectional view showing the other modification example of the cell culture carrier of this invention.
FIG. 5 is a cross-sectional view showing an example of a preferable embodiment of the cell culture vessel of this invention.
FIG. 6 is a cross-sectional view showing a modification example of the cell culture vessel of this invention.
FIG. 7 is a cross-sectional view showing another modification example of the cell culture vessel of this invention.
FIGS. 8A to 8C are cross-sectional views showing an example of a method of replacing a cell culture medium of the cell culture vessel.
FIG. 9 is a cross-sectional view showing a modification example of the method of replacing a cell culture medium of the cell culture vessel.

### DETAILED DESCRIPTION OF THE INVENTION

Hereinafter, the cell culture carrier and the cell culture vessel of this invention will be described in detail.

The cell culture carrier of this invention is a cell culture carrier formed of an anodic oxide film having a plurality of micropores penetrating the film in the thickness direction. An average density of the plurality of micropores is from 1 micropore/µm² to 15,000 micropores/µm², and an average opening ratio of the cell culture carrier is equal to or higher than 51%.

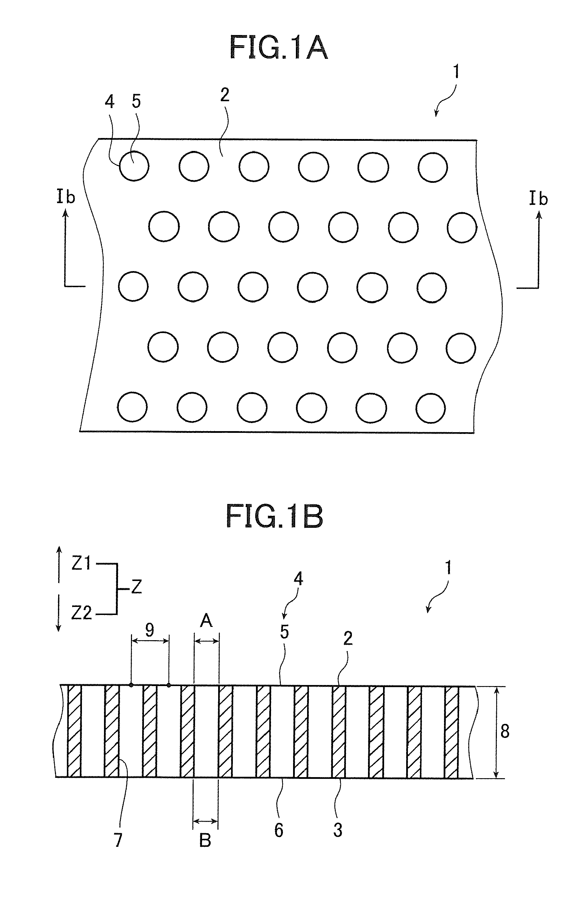

Next, the cell culture carrier of this invention will be described using FIG. 1.

FIG. 1 is a simplified view showing an example of a preferable embodiment of the cell culture carrier of this invention. FIG. 1A is a front view, and FIG. 1B is a cross-sectional view taken along line Ib-Ib of FIG. 1A.

A cell culture carrier 1 of this invention has a plurality of micropores 4 penetrating the cell culture carrier in a thickness direction Z from a front surface 2 to a rear surface 3. The plurality of micropores 4 has a front surface-side opening portion 5 and a rear surface-side opening portion 6. The front surface-side opening portion 5 and the rear surface-side opening portion 6 are connected to each other through an inner circumferential surface 7 of the cell culture carrier 1 that straightly extends in the thickness direction Z. That is, the plurality of micropores 4 is formed such that an average opening diameter A of the front surface-side opening portion 5 is the same as an average opening diameter B of the rear surface-side opening portion 6.

Herein, in the cell culture carrier 1 of this invention, the plurality of micropores 4 having an average density from 1 micropore/µm² to 15,000 micropores/µm² is formed, and an average opening ratio of the cell culture carrier is equal to or higher than 51%.

As described above, in the cell culture carrier 1 of this invention, in order to greatly improve cell viability, the plurality of micropores 4 is formed such that the average opening ratio of the cell culture carrier becomes equal to or higher than 51%. That is, when the cell culture carrier 1 is disposed in a cell culture vessel which will be described later, it is possible to greatly increase the amount of a cell culture medium that flows to the front surface 2 side and the rear surface 3 side through the plurality of micropores 4. Consequentially, by sequentially changing the cell culture medium present around the cells cultured on the front surface 2, viability of the cells can be greatly improved.

It is to be noted that since the amount of the cell culture medium flowing in the plurality of micropores is further increased, the average opening ratio of the cell culture carrier is more preferably equal to or higher than 58%, and even more preferably equal to or higher than 65%. In addition, the average opening ratio of the cell culture carrier is preferably equal to or lower than 90%.

Herein, the average opening ratio can be represented by (opening ratio of front surface + opening ratio of rear surface)/2. The opening ratio of the front surface is represented by "area of front surface opening portion/area of front surface", and the opening ratio of the rear surface is represented by "area of rear surface opening portion/area of rear surface". In order to obtain the area of the opening portion, images of the front surface and rear surface of the cell culture carrier are captured by FE-SEM; for a field of view of 1 µm × 1 µm in the obtained images, binarization is conducted using image analysis software or the like, and a micropore portion and a non-micropore portion are observed; and a geometrical area determined by a method of calculating an equivalent circle diameter of the micropore portion, that is, an area that is assumed to be a two-dimensional plane is obtained. In addition, images of five sites of each of the front surface and rear surface are captured; for the obtained images, the area of the opening portion is obtained in the above manner; and the average of opening ratios calculated from the obtained values is taken as the opening ratio of the front surface and the opening ratio of the rear surface.

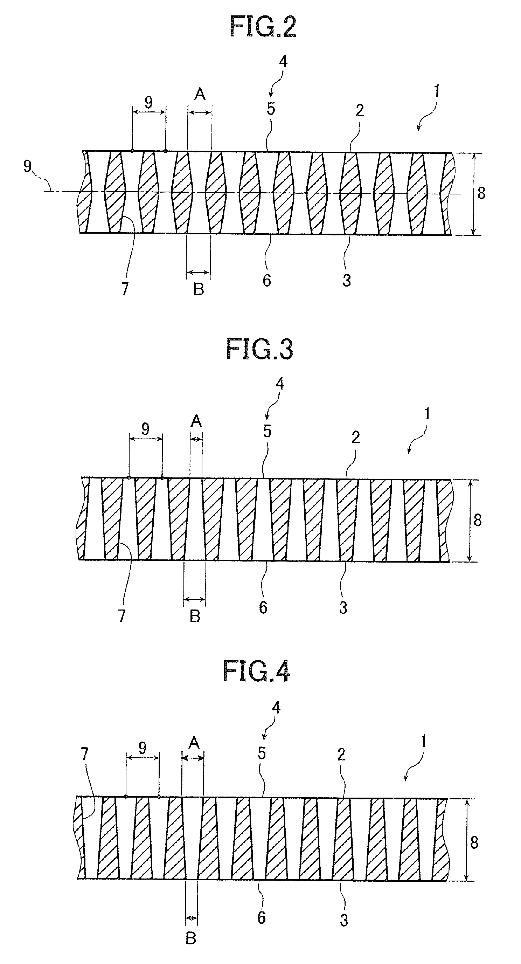

As shown in FIG. 2, the cell culture carrier 1 of this invention can be formed to have a shape in which the average opening diameter A of the front surface-side opening portion 5 of the plurality of micropores 4 is the same as the average opening diameter B of the rear surface-side opening portion 6 thereof, and moreover, the average diameter of the micropores gradually decreases toward an intermediate portion 9 between the front surface 2 and the rear surface 3 from the front surface-side opening portion 5, and also gradually decreases toward the intermediate portion 9 from the rear surface-side opening portion 6. That is, the front surface-side opening portion 5 and the rear surface-side opening portion 6 are connected to each other through an inner circumferential surface 7 of the cell culture carrier 1 that slants inwardly toward the intermediate portion 9.

Moreover, the cell culture carrier of this invention may be a carrier having a shape in which the average opening diameter A of the front surface-side opening portion of the plurality of micropores is different from the average opening diameter B of the rear surface-side opening portion thereof, and the average diameter of the plurality of micropores increases or decreases toward the rear surface-side opening portion from the front surface-side opening portion.

For example, the cell culture carrier 1 of this invention can be formed as shown in FIG. 3 such that the average opening diameter A of the front surface-side opening portion 5 of the plurality of micropores 4 becomes smaller than the average opening diameter B of the rear surface-side opening portion 6 thereof. Furthermore, the front surface-side opening portion 5 and the rear surface-side opening portion 6 are connected to each other through the inner circumferential surface 7 of the cell culture carrier 1 that gently slants such that the diameter of the micropores increases toward the rear surface-side opening portion 6. Consequently, the plurality of micropores 4 has a shape in which the average diameter of the micropores gradually increases toward the rear surface-side opening portion 6 from the front surface-side opening portion 5.

In addition, the cell culture carrier 1 of this invention can be formed as shown in FIG. 4 such that the average opening diameter A of the front surface-side opening portion 5 of the plurality of micropores 4 becomes larger than the average opening diameter B of the rear surface-side opening portion 6 thereof. The front surface-side opening portion 5 and the rear surface-side opening portion 6 are connected to each other through the inner circumferential surface 7 of the cell culture carrier 1 that gently slants such that the diameter of the micropores decreases toward the rear surface-side opening portion 6. Consequently, the plurality of micropores 4 is formed such that the average diameter thereof gradually decreases toward the rear surface-side opening portion 6 from the front surface-side opening portion 5.

Furthermore, the cell culture carrier of this invention can have a shape in which the average diameter of the plurality of micropores increases or decreases stepwise toward the rear surface-side opening portion from the front surface-side opening portion.

As described above, if the cell culture carrier is formed to have a shape in which the average diameter of the plurality of micropores 4 consistently increases or decreases toward the rear surface-side opening portion 6 from the front surface-side opening portion 5, it is possible to allow the cell culture medium to smoothly flow between the front surface 2 side and the rear surface 3 side through the plurality of micropores 4. Accordingly, the cell culture medium is changed by being caused to circulate around the cells cultured on the front surface 2, hence cell viability can be further improved greatly.

In order to allow the cell culture medium to more smoothly flow, as shown in FIGS. 3 and 4, it is preferable for the plurality of micropores to have a shape in which the average diameter thereof gradually increases or decreases toward the rear surface-side opening portion 6 from the front surface-side opening portion 5.

In addition, in order to prevent the opening portion from hindering growth of the cells cultured on the front surface 2, as shown in FIG. 3, it is more preferable for the plurality of micropores 4 to have a shape in which the average diameter thereof gradually increases toward the rear surface-side opening portion 6 from the front surface-side opening portion 5, such that the average opening diameter A of the front surface-side opening portion 5 is further reduced.

Next, the material, dimension, formation, and the like of the cell culture carrier of this invention will be described in detail.

In this invention, the average opening diameter of the plurality of micropores is preferably from 40 nm to 100 nm.

Moreover, as shown in FIGS. 3 and 4, when the plurality of micropores is formed to have a shape in which the average diameter thereof increases or decreases toward the rear surface-side opening portion from the front surface-side opening portion, between the average opening diameter A of the front surface-side opening portion and the average opening diameter B of the rear surface-side opening portion of the plurality of micropores, the smaller average opening diameter is preferably from 40 nm to 80 nm. Furthermore, between the average opening diameter A of the front surface-side opening portion and the average opening diameter B of the rear surface-side opening portion of the plurality of micropores, the larger average opening diameter is preferably from 65 nm to 100 nm.

If the average opening diameter is within the above range, it is possible to further increase the amount of cell culture medium flowing to the front surface side and the rear surface side of the cell culture carrier through the plurality of micropores, and to further improve cell viability. In addition, if the average opening diameter is within the above range, it is possible to inhibit growth of cells, which are cultured on the front surface of the cell culture carrier, from being hindered.

A center-to-center distance (portion indicated by reference number 9 in FIG. 1, also referred to as "pitch") between the respective micropores adjacent to each other is preferably from 10 nm to 500 nm, more preferably from 30 nm to 400 nm, and even more preferably from 50 nm to 300 nm. If the pitch is within the above range, it is possible to arrange the plurality of micropores in a balanced manner, and to further improve the cell viability by evenly supplying the cell culture medium.

Herein, the average opening diameter is a value obtained by capturing an image (20,000× magnification) of the surface by FE-SEM, measuring an opening diameter of each of the micropores present in a field of view of 1 µm × 1 µm in the image, and calculating the average thereof. The center-to-center distance is a value obtained by capturing images (20,000x magnification) of five sites of the surface of the cell culture carrier by FE-SEM, measuring a distance between centers of the respective micropores present in a field of view of 1 µm × 1 µm in each of the images of the surface, and calculating the average of the center-to-center distance of the micropores obtained from the images of five sites of the surface.

In order to further increase the amount of flowing cell culture medium, the average density of the plurality of micropores is preferably from 2 micropores/µm² to 1,000 micropores/µm², and more preferably from 3 micropores/µm² to 300 micropores/µm².

Herein, the average density is a value obtained by capturing an image (20,000× magnification) of the surface by FE-SEM, counting the number of micropores present in a field of view of 1 µm × 1 µm in the image to obtain density of the micropores, and calculating the average of the thus obtained density for five sites of the field of view of 1 µm × 1 µm.

In order to further increase the amount of flowing cell culture medium, the thickness (indicated by reference number 8 in FIG. 1B) of the cell culture carrier is preferably equal to or smaller than 300 µm, more preferably equal to or smaller than 200 µm, and even more preferably equal to or smaller than 150 µm.

Moreover, in order to further increase the amount of flowing cell culture medium, an aspect ratio (average length/average opening diameter) of the plurality of micropores is preferably equal to or lower than 5,000, more preferably equal to or lower than 3,000, and even more preferably equal to or lower than 2,000.

As shown in FIGS. 3 and 4, when the plurality of micropores has a shape in which the average diameter thereof increases or decreases toward the rear surface-side opening portion from the front surface-side opening portion, between the average opening diameter A of the front surface-side opening portion and the average opening diameter B of the rear surface-side opening portion of the plurality of micropores, the larger average opening diameter is greater than the smaller average opening diameter, preferably by 1.05-fold to 10.0-fold, more preferably by 1.1-fold to 5.0-fold, and even more preferably by 1.15-fold to 3.0-fold.

If the ratio between the average opening diameter A and the average opening diameter B is within the above range, it is possible to allow the cell culture medium to more smoothly flow between the front surface side and the rear surface side through the plurality of micropores and to further improve the cell viability.

Next, a production method of the cell culture carrier of this invention will be described in detail.

Although the production method of the cell culture carrier of this invention is not particularly limited, examples of the production method include a method of forming a cell culture carrier by at least performing anodizing treatment (hereinafter, also referred to as "anodizing treatment (A)") in which an oxide film having micropores is formed by anodizing an aluminum substrate, separating treatment (hereinafter, also referred to as "separating treatment (B)") in which the oxide film is separated from the aluminum substrate by removing the aluminum substrate after the anodizing treatment, and penetrating treatment (hereinafter, also referred to as "penetrating treatment (C)") in which the micropores of the oxide film separated by the separating treatment are made to penetrate the oxide film, in this order.

### [Aluminum substrate]

As the aluminum substrate subjected to each of the treatments which will be described later, those described in paragraphs [0010] to [0012] of JP 2010-226975 A can be used. Thermal treatment, degreasing treatment, and mirror finishing treatment can also be performed in the same manner as each of the treatments described in paragraphs [0013] to [0023] of JP 2010-226975 A.

### [Anodizing treatment (A)]

The anodizing treatment (A) is treatment in which an oxide film having micropores is formed on the surface of an aluminum substrate by anodizing the aluminum substrate. As the anodizing treatment, conventionally known methods can be used. However, from the viewpoint of arranging the micropores with a high degree of regularity, it is preferable to use self-regularization process or constant voltage treatment.

Herein, the anodizing treatment can be performed in the same manner as each of the treatments described in paragraphs [0024] to [0071] of JP 2010-226975 A.

### [Separating treatment (B)]

The separating treatment (B) is treatment in which the anodic oxide film is separated from the aluminum substrate by removing the aluminum substrate after the anodizing treatment (A). For removing the aluminum substrate, for example, the aluminum substrate having undergone the anodizing treatment is brought into contact with a treatment solution which dissolves aluminum but does not dissolve alumina, and in this manner, the anodic oxide film from which the aluminum substrate has been removed can be obtained.

Herein, the separating treatment can be performed in the same manner as each of the treatments described in [0072] to [0076] of JP 2010-226975 A.

### [Penetrating treatment (C)]

The penetrating treatment (C) is treatment in which the micropores of the anodic oxide film separated by the separating treatment (B) are made to penetrate the anodic oxide film. By the penetrating treatment, a cell culture carrier having an average opening ratio of equal to or higher than 51% can be obtained.

In the penetrating treatment, the anodic oxide film obtained by the separating treatment is brought into contact with an aqueous acid solution or an aqueous alkali solution, such that the anodic oxide film is partially dissolved.

Herein, in the penetrating treatment, it is preferable to bring the anodic oxide film into contact with an aqueous acid solution or an aqueous alkali solution, which has concentration lower than that of an aqueous acid solution or an aqueous alkali solution in the penetrating treatment (C) described in JP 2010-226975 A, over a long time. If the anodic oxide film is brought into contact with such a solution over a long time, the penetrating treatment proceeds calmly, and the average opening ratio of equal to or higher than 51% can be reliably accomplished.

Herein, in the penetrating treatment (C), by varying the treatment time, treatment temperature, concentration of an acid solution or an alkali solution used for the treatment, and the like, the average opening ratio of the cell culture carrier can be controlled.

When an aqueous acid solution is used for the penetrating treatment, it is preferable to use an aqueous solution of an inorganic acid such as sulfuric acid, phosphoric acid, nitric acid, or hydrochloric acid or to use an aqueous solution of a mixture of these acids. The concentration of the aqueous acid solution is preferably 0.1% by mass to 0.9% by mass, since the average opening ratio of equal to or higher than 51% is more reliably obtained. For the same reason, the temperature of the aqueous acid solution is preferably 15°C to 35°C.

When the aqueous alkali solution is used for the penetrating treatment, it is preferable to use an aqueous solution of at least one kind of alkali selected from a group consisting of sodium hydroxide, potassium hydroxide, and lithium hydroxide. The concentration of the aqueous alkali solution is preferably 0.01% by mass to 0.09% by mass, since the average opening ratio of equal to or higher than 51% is more reliably obtained. For the same reason, the temperature of the aqueous alkali solution is preferably 10°C to 30°C.

Specifically, for example, a 5 g/L of aqueous phosphoric acid solution at 30°C, a 0.2 g/L aqueous sodium hydroxide solution at 15°C, or a 0.2 g/L aqueous potassium hydroxide solution at 15°C is preferably used.

The anodic oxide film is dipped in the aqueous acid solution or the aqueous alkali solution preferably for 121 minutes to 200 minutes, more preferably for 130 minutes to 190 minutes, and even more preferably for 135 minutes to 180 minutes, since the average opening ratio of equal to or higher than 51% is more reliably obtained.

After the penetrating treatment, the anodic oxide film is subjected to washing treatment using water. In order to inhibit the opening diameter of micropores from changing due to hydration, it is preferable to perform the washing treatment using water at a temperature equal to or lower than 30°C.

Moreover, after the separating treatment, at any point in time before or after the penetrating treatment, heating treatment in which the oxide coating formed by the anodizing treatment is heated for at least 10 minutes at a temperature equal to or higher than 50°C can be performed.

As shown in FIGS. 3 and 4, when the plurality of micropores has a shape in which the average diameter thereof increases or decreases toward the rear surface-side opening portion from the front surface-side opening portion, after the penetrating treatment (C), it is preferable to perform opening-enlarging treatment (hereinafter, also referred to as "opening-enlarging treatment (D)") in which the opening diameter of the micropores, which have been made to penetrate the anodic oxide film by the penetrating treatment, of the front surface-side opening portion or the rear surface-side opening portion is enlarged.

### [Opening-enlarging treatment (D)]

In the opening-enlarging treatment (D), the front surface portion or the rear surface portion of the anodic oxide film obtained by the penetrating treatment is brought into contact with an aqueous acid solution or an aqueous alkali solution. The method of bringing the anodic oxide film into contact with such a solution is not particularly limited, and examples of the method include a dipping method and a spraying method. Between these, the dipping method is preferable.

When the aqueous acid solution is used for the opening-enlarging treatment, it is preferable to use an aqueous solution of an inorganic acid such as sulfuric acid, phosphoric acid, nitric acid, or hydrochloric acid or to use an aqueous solution of a mixture of these acids. The concentration of the aqueous acid solution is preferably 0.1% by mass to 1% by mass, and the temperature of the aqueous acid solution is preferably 15°C to 30°C.

When the aqueous alkali solution is used for the opening-enlarging treatment, it is preferable to use an aqueous solution of at least one kind of alkali selected from a group consisting of sodium hydroxide, potassium hydroxide, and lithium hydroxide. The concentration of the aqueous alkali solution is preferably 0.01% by mass to 1% by mass, and the temperature of the aqueous alkali solution is preferably 15°C to 30°C.

The anodic oxide film is dipped in the aqueous acid solution or the aqueous alkali solution, preferably for 0.5 minutes to 30 minutes, more preferably for 1 minute to 25 minutes, and even more preferably for 3 minutes to 20 minutes.

Hereinafter, the cell culture vessel of this invention will be described in detail.

### [Cell culture vessel]

The cell culture vessel of this invention is a cell culture vessel including at least one culture well that has a culture chamber accommodating a cell culture medium and the aforementioned cell culture carrier that has a front surface, to which cells adhere, positioned inside the culture chamber and is disposed such that the cell culture medium fills the cell culture carrier from the front surface to the rear surface of the cell culture carrier.

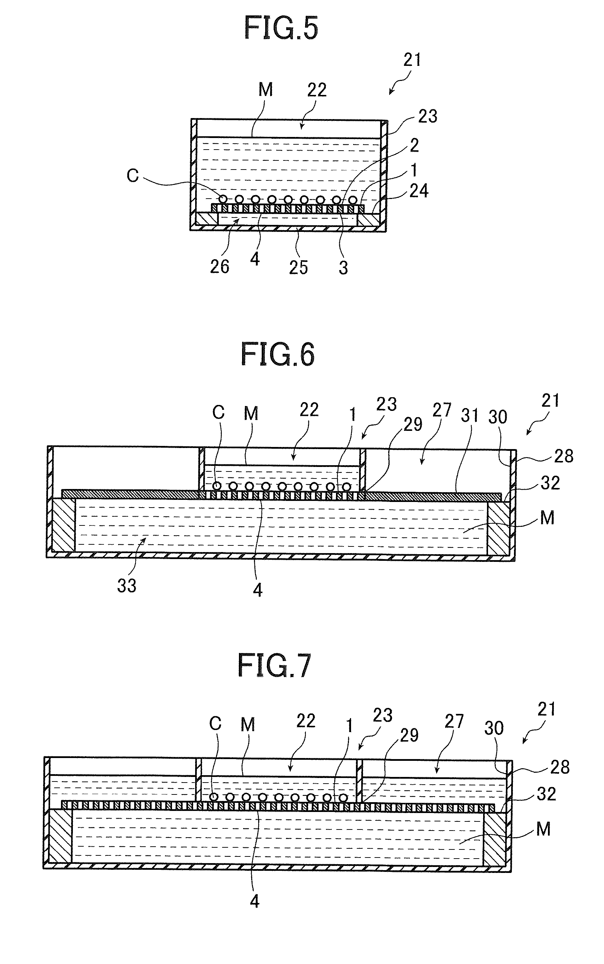

FIG. 5 is a schematic cross-sectional view showing an example of a preferable embodiment of a cell culture vessel 21 of this invention.

The cell culture vessel 21 has the shape of a cylinder of which the top is opened. The cell culture vessel 21 includes a culture well 23 that has a culture chamber 22 accommodating a cell culture medium M inside the chamber and the cell culture carrier 1 that is disposed such that the front surface 2 and the rear surface 3 are positioned inside the culture chamber 22. A cylindrical supporter 24 is disposed inside the culture chamber 22. The supporter 24 supports the cell culture carrier 1 from below, and accordingly, a gap 26 filled with the cell culture medium M is formed between the rear surface 3 of the cell culture carrier 1 and a bottom plate 25 of the culture chamber 22.

As described above, the cell culture carrier 1 has the plurality of micropores 4. Accordingly, the cell culture medium M can be brought into contact with a cell C, which is cultured in a state of adhering onto the front surface 2 of the cell culture carrier 1, not only from above the cell but also from below the cell. Consequentially, growth of the cell can be accelerated. Moreover, as described above, the average opening ratio of the cell culture carrier 1 is equal to or higher than 51%. Therefore, it is possible to greatly increase the amount of cell culture medium flowing to the front surface 2 side and the rear surface 3 side through the plurality of micropores 4 and to greatly improve the cell viability.

Moreover, the cell culture vessel of this invention can be configured such that the vessel further includes an accommodation portion that has an accommodation chamber accommodating the cell culture medium in an amount greater than that of the cell culture medium accommodated in the culture chamber; the cell culture carrier configures at least a portion of the bottom plate of the culture well; the culture well is disposed such that the bottom plate is positioned inside the accommodation chamber; and the culture chamber is in communication with the accommodation chamber through the plurality of micropores.

For example, as shown in FIG. 6, the cell culture vessel can includes the culture well 23 that has the shape of a cylinder of which the top is opened, the bottom plate configured with the aforementioned cell culture carrier 1, and the culture chamber 22 accommodating the cell culture medium M inside the chamber; and an accommodation portion 28 that has the shape of a cylinder of which the top is opened and an accommodation chamber 27 accommodating the cell culture medium M, which is in an amount greater than that of the cell culture medium M accommodated in the culture chamber 22 of the culture well 23, inside the chamber. In this vessel, the culture well 23 can be positioned inside the accommodation chamber 27 of the accommodation portion 28. The culture well 23 has an expansion portion 31 which is in the form of a disc extending toward a side 30 of the accommodation portion 28 from a side edge portion 29 of the cell culture carrier 1. The accommodation portion 28 has a supporter 32, which supports the expansion portion 31 from below, inside the accommodation chamber 27. Consequentially, a gap 33 is formed between the bottom surface of the accommodation portion 28 and the rear surface 3 of the cell culture carrier 1, and the cell culture medium M accommodated in the accommodation chamber 27 can fill the gap 33. The culture chamber 22 of the culture well 23 is in communication with the accommodation chamber 27 of the accommodation portion 28 through the plurality of micropores 4. Therefore, the cell culture medium M accommodated in the culture chamber 22 and the cell culture medium M accommodated in the accommodation chamber 27 can be exchanged with each other through the plurality of micropores 4.

Furthermore, as shown in FIG. 7, it is preferable for the expansion portion 31 to be formed of an anodic oxide film having a plurality of micropores penetrating the film in the thickness direction and to be disposed in the vessel integrally with the cell culture carrier 1. That is, it is preferable for the bottom plate 25 and the expansion portion 31 of the culture well 23 to be configured with the aforementioned cell culture carrier 1.

As described above, the average opening ratio of the cell culture carrier 1 is equal to or higher than 51%. Accordingly, it is possible to greatly increase the amount of the cell culture medium flowing to the culture chamber 22 side and the accommodation chamber 27 side through the plurality of micropores 4. It is generally known that during cell culturing, due to lactic acid and the like generated from the cell C cultured, the cell culture medium M deteriorates, and this leads to decrease in viability of the cell C. In the cell culture vessel 21 of this invention, the amount of the cell culture medium M flowing to the culture chamber 22 side and the accommodation chamber 27 side is increased such that the cell culture medium M is sequentially changed around the cell C cultured on the front surface 2 of the cell culture carrier 1. Therefore, the viability of the cell C can be greatly increased.

Moreover, generally, during cell culturing, in order to prevent the cell viability from greatly decreasing due to marked deterioration of the cell culture medium M, the cell culture medium M in the culture chamber 22 needs to be periodically replaced. In the cell culture vessel 21 of this invention, the amount of the cell culture medium M flowing to the culture chamber 22 and the accommodation chamber 27 is increased, and accordingly, deterioration of the cell culture medium M accommodated in the culture chamber 22 is inhibited. Therefore, the number of times the cell culture medium M is replaced can be reduced. Furthermore, as described in the cell culture medium-replacing method which will be described later, it is possible to replace the cell culture medium M while reducing a load applied to the cell C, and as a result, decrease in the cell viability can be further inhibited.

The cells cultured in the cell culture vessel of this invention are not particularly limited, and for example, it is possible to use normal cells such as mesenchymal cells, hepatocytes, fibroblasts, endothelial cells, nerve cells, myocardial cells, glial cells, corneal epithelial cells, chondrocytes, osteoblasts, and adipocytes. It is also possible to use abnormal cells such as cancer-derived cell strains (for example, HepG2 and HuH-7), immortalized cells (for example, HHY41, NKNT-3, and Fa2N-4 strains), and cells showing chromosomal aberration. Moreover, iPS cells, ES cells, and the like can be used.

As the cell culture medium used in the cell culture vessel of this invention, it is possible to use a cell culture medium appropriately prepared according to the type of cell to be cultured.

Hereinafter, regarding the cell culture vessel of this invention shown in FIG. 7, a cell culture medium-replacing method will be described in detail.

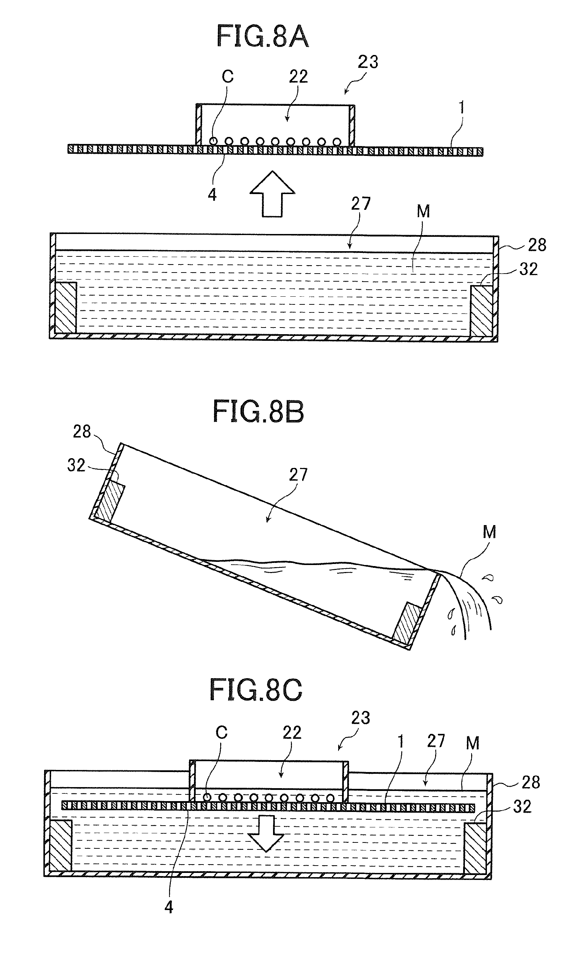

First, when the cell C is cultured in the state shown in FIG. 7, and the cell culture medium M deteriorates, as shown in FIG. 8A, the culture well 23 is taken out of the accommodation chamber 27 of the accommodation portion 28. At this time, the cell culture medium M in the culture chamber 22 flows into the accommodation chamber 27 through the plurality of micropores 4 of the cell culture carrier 1 configuring the bottom plate of the culture well 23. That is, the entirety of the cell culture medium M in the cell culture vessel 21 is accommodated in the accommodation chamber 27.

Subsequently, as shown in FIG. 8B, after the cell culture medium M having been accommodated in the accommodation chamber 27 is removed, a new cell culture medium M is supplied into the accommodation chamber 27. Thereafter, as shown in FIG. 8C, the culture well 23 is accommodated in the accommodation chamber 27 of the accommodation portion 28. At this time, as the culture well 23 gradually sinks into the cell culture medium M of the accommodation chamber 27, the cell culture medium M flows into the culture chamber 22 from the accommodation chamber 27 through the plurality of micropores 4 of the cell culture carrier 1, and at a point in time when the culture well 23 comes into contact with the supporter 32, the culture chamber 22 is filled with the cell culture medium M.

It is generally known that when the cell culture medium M in the culture chamber 22 is replaced using a tool such as a pipette, the cell culture medium M is powerfully supplied from the pipette, or alternatively, the tip of the pipette comes into direct contact with the cell, and consequentially, the cell C is damaged, resulting in decrease in viability of the cell C. In the cell culture vessel of this invention shown in FIG. 7, as described above, it is possible to calmly replace the cell culture medium M in the culture chamber 22 without using a tool such as a pipette, and accordingly, decrease in the viability of the cell C can be inhibited. In the cell culture vessel shown in FIG. 6, the cell culture medium M can be replaced in the same manner as above, and accordingly, decrease in the viability of the cell C can be inhibited.

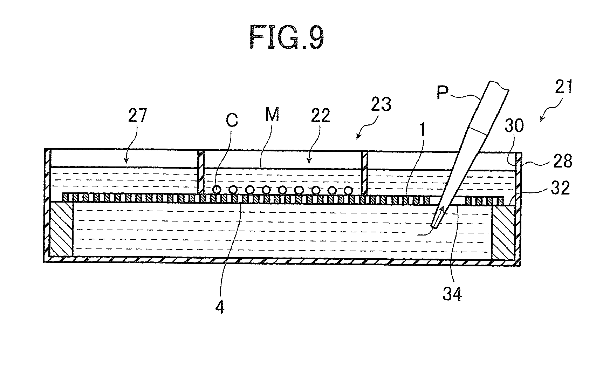

As shown in FIG. 9, in a portion of the cell culture carrier 1 configuring the expansion portion of the culture well 23, an insertion hole 34 for inserting a pipette P may be formed, such the cell culture medium M accommodated in the accommodation chamber 27 can be replaced through the insertion hole 34, and at the same time, the cell culture medium M accommodated in the culture chamber 22 can be replaced.

As described above, since the pipette P is not directly inserted into the culture chamber 22, it is possible to calmly replace the cell culture medium M.

### EXAMPLES

Hereinafter, this invention will be described specifically based on examples. However, this invention is not limited to the examples.

### (Example 1)

### (1) Electrolytic polishing treatment

A high-purity aluminum substrate (manufactured by Sumitomo Light Metal Industries, Ltd., purity of 99.999% by mass, thickness of 0.4 mm) was cut into an area of 10 cm × 10 cm and subjected to electrolytic polishing treatment by using an electrolytic polishing solution having the following composition, under conditions of a voltage of 10 V and a solution temperature of 65°C. A carbon electrode was used as a cathode, and GP-250-30R (manufactured by TAKASAGO LTD.) was used as a power source.

### (Composition of electrolytic polishing solution)

- 85% by mass phosphoric acid (reagent manufactured by Wako Pure Chemical Industries, Ltd.) 1,320 mL
- Pure water 80 mL
- Sulfuric acid 600 mL

### (2) Anodizing treatment

A sample obtained after the electrolytic polishing treatment performed as above was subjected to anodizing treatment for 25 minutes by using an electrolytic solution of 0.50 mol/L oxalic acid, under conditions of a voltage of 40.0 V, a solution temperature of 15°C, and a solution flow rate of 3.0 m/min. Moreover, the sample obtained after the anodizing treatment was subjected to film-removing treatment in which the sample was dipped in an aqueous mixed solution of 0.5 mol/L phosphoric acid for 20 minutes at 40°C. This treatment was repeated 4 times.

Furthermore, the sample was subjected to re-anodizing treatment for 15 hours by using an electrolytic solution of 0.5 mol/L oxalic acid, under re-anodizing treatment conditions of a voltage of 41.7 V, a solution temperature of 15°C, and a solution flow rate of 3.0 m/min. In addition, the sample was subjected to the film-removing treatment in which the sample was dipped in an aqueous mixed solution of 0.5 mol/L phosphoric acid for 20 minutes at 40°C. As a result, on the surface of the aluminum substrate, an anodic oxide film in which straight tube-like micropores were arranged in the form of honeycomb was formed.

In both the anodizing treatment and re-anodizing treatment, a stainless steel electrode was used as a cathode, and GP0110-30R (manufactured by TAKASAGO LTD.) was used as a power source. In addition, NeoCool BD36 (manufactured by YAMATO SCIENTIFIC CO., LTD.) was used as a cooling apparatus, and Pair Stirrer PS-100 (manufactured by EYELA TOKYO RIKAKIKAI CO., LTD.) was used as a stirring and heating apparatus.

### (3) Separating treatment

The sample obtained after the anodizing treatment performed as above was dipped in an aqueous mixed solution consisting essentially of 20% by mass hydrochloric acid and 0.1 mol/L cupric chloride, for 20 minutes at 10°C. In this manner, the aluminum substrate was dissolved and removed, and a microstructure formed of an anodic oxide film having micropores was prepared.

### (4) Penetrating treatment

The sample obtained after the separating treatment performed as above was dipped in a KCl solution as a pH buffer solution for 10 minutes such that the KCl solution sufficiently flowed into the micropores. Thereafter, only the side of a barrier layer was dipped in 0.01 M KOH for 140 minutes at 15°C so as to remove the bottom portion of the anodic oxide film, thereby preparing a cell culture carrier formed of an anodic oxide film having micropores of which the rear surface opening diameter was enlarged.

### <Shape analysis of cell culture carrier>

For the cell culture carrier obtained after the penetrating treatment performed as above, images (20,000× magnification) of five sites of the front surface and rear surface of the carrier and images (20,000× magnification) of cross-section of five sites of the carrier were captured by FE-SEM.

The average opening diameter A was obtained by measuring the diameter of all of the micropores present in a field of view of 1 µm × 1 µm in the images of five sites of the front surface and calculating the average thereof.

The average opening diameter B was obtained by measuring the diameter of all of the micropores present in a field of view of 1 µm × 1 µm in the images of five sites of the rear surface and calculating the average thereof.

The center-to-center distance was obtained by measuring the center-to-center distances of all of the micropores present in a field of view of 1 µm × 1 µm in the images of five sites of the front surface and the images of five sites of the rear surface, and calculating the average thereof.

Herein, the front surface refers to a surface in which formation of the micropores is started in the anodizing treatment, and the rear surface refers to a surface from which the aluminum substrate is removed in the separating treatment.
- Thickness of cell culture carrier: 150 µm
- Depth of micropores: 150 µm
- Average opening diameter A of front surface: 75 nm
- Average opening diameter B of rear surface: 75 nm
- Center-to-center distance of micropores: 100 nm (5) Sterilizing treatment

The cell culture carrier obtained as above was subjected to sterilizing treatment in which ultrasonic cleaning was performed for 5 minutes on the carrier by using ethanol, and then the carrier was dried in an oven.

### (6) Cell culturing process

After the sterilization, cells were cultured using the cell culture carrier cooled to room temperature.
(a) Used cells: BAE (Bovine Aortic Endothelial cells)
(b) Used culture medium: Eagle minimum medium, 10% fetal bovine serum
(c) Pretreatment: A petri dish having a height of 10 mm and an inner diameter of 35 mm was prepared, and a cylindrical supporter configured with polystyrene that had a thickness of 1 mm, an outer diameter of 35 mm, and a height of 5 mm was disposed in the petri dish. Thereafter, the cell culture medium was filled in the petri dish up to a height of 8 mm, and then the cell culture carrier was sunk into the cell culture medium and disposed on the supporter. Subsequently, on the front surface of the cell culture carrier, a side of the culture well 23 configured with polystyrene that had a thickness of 1 mm, an outer diameter of 10 mm, and a height of 5 mm was disposed.
(d) Cell seeding: Cells that had been cultured beforehand were collected by trypsin treatment, and the cell concentration was regulated to be 40,000 cells/mL. After the medium in the culture chamber of the culture well was discarded, a cell sap of which the cell count had been beforehand regulated to be 7,000 cells/cm² was seeded into the culture chamber.
(e) Culturing: The cells were cultured for 3 days at 37°C by using a CO₂ incubator.

### (Example 2)

A cell culture carrier was prepared, and cells were cultured, in the same manner as in Example 1, except that the time taken for treating the carrier with 0.01 M KOH was changed to 150 minutes in the (4) penetrating treatment of Example 1. The results obtained by checking the shape of the cell culture carrier are shown below.
- Thickness of cell culture carrier: 150 µm
- Depth of micropores: 150 µm
- Average opening diameter A of front surface: 81 nm
- Average opening diameter B of rear surface: 81 nm
- Center-to-center distance of micropores: 100 nm

### (Example 3)

A cell culture carrier was prepared, and cells were cultured, in the same manner as in Example 1, except that the time taken for treating the carrier with 0.01 M KOH was changed to 160 minutes in the (4) penetrating treatment of Example 1. The results obtained by checking the shape of the cell culture carrier are shown below.
- Thickness of cell culture carrier: 150 µm
- Depth of micropores: 150 µm
- Average opening diameter A of front surface: 90 nm
- Average opening diameter B of rear surface: 90 nm
- Center-to-center distance of micropores: 100 nm

### (Example 4)

A cell culture carrier was prepared, and cells were cultured, in the same manner as in Example 1, except that the time taken for treating the carrier with 0.01 M KOH was changed to 170 minutes in the (4) penetrating treatment of Example 1. The results obtained by checking the shape of the cell culture carrier are shown below.
- Thickness of cell culture carrier: 150 µm
- Depth of micropores: 150 µm
- Average opening diameter A of front surface: 95 nm
- Average opening diameter B of rear surface: 95 nm
- Center-to-center distance of micropores: 100 nm

### (Example 5)

A cell culture carrier was prepared in the same manner as in Example 2. Thereafter, cells were cultured in the same manner as in Example 2, except that the cells and culture medium used in the cell culturing process were changed as below.
(a) Used cells: rat hepatocytes
(b) Used culture medium: DMEM medium

### (Example 6)

A cell culture carrier was prepared in the same manner as in Example 2. Thereafter, cells were cultured in the same manner as in Example 2, except that the cells and culture medium used in the cell culturing process were changed as below.
(a) Used cells: HepG2 cells (human embryoma-derived cells)
(b) Used culture medium: William's E medium, 10% fetal bovine serum

### (Example 7)

A cell culture carrier was prepared in the same manner as in Example 2. Thereafter, cells were cultured in the same manner as in Example 2, except that the cells and culture medium used in the cell culturing process were changed as below.
(a) Used cells: HuH7 cells (human hepatoma-derived cells)
(b) Used culture medium: William's E medium, 10% fetal bovine serum

### (Example 8)

A cell culture carrier was prepared in the same manner as in Example 2. Thereafter, cells were cultured in the same manner as in Example 2, except that the cells and culture medium used in the cell culturing process were changed as below.
(a) Used cells: RIN-5F cells (rat Langerhans islet-derived cells)
(b) Used culture medium: RPMI-1640 medium, 10% fetal bovine serum

### (Example 9)

A cell culture carrier was prepared in the same manner as in Example 2. Thereafter, cells were cultured in the same manner as in Example 2, except that the cells and culture medium used in the cell culturing process were changed as below.
(a) Used cells: 129SV cells (mouse ES cells)
(b) Used culture medium: medium for ES cells

### (Comparative example 1)

A cell culture carrier was prepared as below, with reference to the preparation method for membrane 2 described in paragraph [0104] of JP 2010-226975 A and the preparation method of Comparative example 1 described in paragraph [0127] of the same document.

### (1) Electrolytic polishing treatment

Electrolytic polishing treatment was performed in the same manner as in Example 1, except that a high-purity aluminum substrate having purity of 99.99% by mass was used.

### (2) Anodizing treatment

A sample obtained after the electrolytic polishing treatment performed as above was subjected to anodizing treatment for 2.5 hours by using an electrolytic solution of 0.50 mol/L oxalic acid, under conditions of a voltage of 40.0 V, a solution temperature of 15°C, and a solution flow rate of 3.0 m/min. Moreover, the sample obtained after the anodizing treatment was subjected to film-removing treatment in which the sample was dipped in an aqueous mixed solution of 0.5 mol/L phosphoric acid for 20 minutes at 40°C. This treatment was repeated 4 times.

Furthermore, the sample was subjected to re-anodizing treatment for 10 hours by using an electrolytic solution of 0.5 mol/L oxalic acid, under re-anodizing treatment conditions of a voltage of 41.7 V, a solution temperature of 15°C, and a solution flow rate of 3.0 m/min. In addition, the sample was subjected to the film-removing treatment in which the sample was dipped in an aqueous mixed solution of 0.5 mol/L phosphoric acid for 20 minutes at 40°C. As a result, on the surface of aluminum substrate, an anodic oxide film in which straight tube-like micropores were arranged in the form of honeycomb was formed.

### (3) Separating treatment

Separating treatment was performed in the same manner as in Example 1.

### (4) Penetrating treatment

The sample obtained after the separating treatment performed as above was dipped in a KCl solution as a pH buffer solution for 10 minutes such that the KCl solution sufficiently flowed into the micropores. Thereafter, only the side of a barrier layer was dipped in 0.1 M KOH for 4 minutes at 30°C so as to remove the bottom portion of the anodic oxide film, thereby preparing a cell culture carrier formed of an anodic oxide film having micropores of which the diameter is consistent from the front surface-side opening portion to the rear surface-side opening portion.

Paragraph [0104] of JP 2010-226975 A describes "only the side of a barrier layer was dipped in 0.1 M KOH for 45 minutes at 30°C so as to remove the bottom portion of the anodic oxide film" in (4) Process of removing aluminum and performing penetrating treatment. However, the time taken for such treatment is considered to be too long to obtain the shape of a microstructure checked by capturing images (for example, a microstructure having an average opening diameter of through holes of 30 nm). Actually, when the sample obtained after the separating treatment was dipped in 0.1 M KOH for 45 minutes at 30°C, most of the sample was dissolved. Therefore, the description of JP 2010-226975 A is considered to be an erroneous description which may be corrected to "dipped in 0.1 M KOH for 4 minutes at 30°C".

The results obtained by checking the shape of the cell culture carrier are shown below.
- Thickness of cell culture carrier: 100 µm
- Depth of micropores: 100 µm
- Average opening diameter A of front surface: 30 nm
- Average opening diameter B of rear surface: 30 nm
- Center-to-center distance of micropores: 100 nm (6) Cell culturing process

Cells were cultured in the same manner as in Example 1.

### (Comparative example 2)

A cell culture carrier was prepared, and cells were cultured, in the same manner as in Comparative example 1, except that the time taken for treating the carrier with 0.1 M KOH was changed to 5 minutes in the (4) penetrating treatment of Comparative example 1. The results obtained by checking the shape of the cell culture carrier are shown below.
- Thickness of cell culture carrier: 150 µm
- Depth of micropores: 150 µm
- Average opening diameter A of front surface: 43 nm
- Average opening diameter B of rear surface: 43 nm
- Center-to-center distance of micropores: 100 nm

### (Comparative example 3)

A cell culture carrier was prepared, and cells were cultured, in the same manner as in Comparative example 1, except that the time taken for treating the carrier with 0.1 M KOH was changed to 9 minutes in the (4) penetrating treatment of Comparative example 1. The results obtained by checking the shape of the cell culture carrier are shown below.
- Thickness of cell culture carrier: 150 µm
- Depth of micropores: 150 µm
- Average opening diameter A of front surface: 68 nm
- Average opening diameter B of rear surface: 68 nm
- Center-to-center distance of micropores: 100 nm

The average opening ratio of both surfaces of the prepared cell culture carrier was obtained by calculating a value of (opening ratio of front surface + opening ratio of rear surface)/2. Herein, the opening ratio of front surface was obtained by capturing images of five sites of the front surface of the cell culture carrier by FE-SEM; for a field of view of 1 µm × 1 µm in each of the obtained images, obtaining an area of the front surface opening portion and an area of the range of the field of view; calculating an opening ratio by a formula of "area of front surface opening portion/area of range of field of view"; and calculating the average of the opening ratios of the five sites. The opening ratio of the rear surface was obtained by capturing images of five sites of the rear surface of the cell culture carrier by FE-SEM; for a field of view of 1 µm × 1 µm in each of the obtained images, obtaining an area of the rear surface opening portion and an area of the range of the field of view; calculating an opening ratio by a formula of "area of rear surface opening portion/area of range of field of view"; and calculating the average of the opening ratios of the five sites. The results are shown in the following Table 1.

### (Evaluation method)

By using an optical microscope, 100 cells were randomly selected, and the number of surviving cells among the selected cells was counted, thereby calculating the cell viability. If the cell viability was equal to or higher than 92.5%, the cell viability was evaluated to be A; if the cell viability was equal to or higher than 90% but less than 92.5%, it was evaluated to be B; and if the cell viability was less than 90%, it was evaluated to be C. The results are shown in the following Table 1.

[Table 1]

**Table 1**

| | Cell culture carrier | | | Cell culturing | Evaluation |
|---|---|---|---|---|---|
| | Front surface-side opening portion | Rear surface-side opening portion | Average opening ratio of both surfaces (%) | Type of cell | Cell viability (%) |
| | Average opening diameter A (nm) | Average opening diameter B (nm) | | | |
| Example 1 | 75 | 75 | 51 | BAE | B |
| Example 2 | 81 | 81 | 60 | BAE | A |
| Example 3 | 90 | 90 | 73 | BAE | A |
| Example 4 | 95 | 95 | 82 | BAE | A |
| Example 5 | 81 | 81 | 60 | Rat hepatocyte | A |
| Example 6 | 81 | 81 | 60 | HePG2 | A |
| Example 7 | 81 | 81 | 60 | HuH7 | A |
| Example 8 | 81 | 81 | 60 | RIN-5F | A |
| Example 9 | 81 | 81 | 60 | 129SV | A |
| Comparative Example 1 | 30 | 30 | 8 | BAE | C |
| Comparative Example 2 | 43 | 43 | 17 | BAE | C |
| Comparative Example 3 | 68 | 68 | 42 | BAE | C |

From the results shown in Table 1, it was understood that improvement of cell viability becomes greater in Examples 1 to 9 in which the average opening ratio of the cell culture carrier is equal to or higher than 51%, than in Comparative examples 1 to 3 in which the average opening ratio is less than 51%.

Moreover, it was understood that cell viability is further improved in Examples 2 to 9 in which the average opening ratio of the cell culture carrier is equal to or higher than 58%, than in Example 1 in which the average opening ratio of the cell culture carrier is less than 58%.

Furthermore, any of Examples 2 and 5 to 9 in which different types of cells were cultured exhibited high cell viability, and consequentially, it was understood that cell viability can be greatly improved regardless of the type of cells.

## Claims

1. A cell culture carrier comprising:
an anodic oxide film having a plurality of micropores penetrating the anodic oxide film in a thickness direction,
wherein an average density of the plurality of micropores is from 1 micropore/µm² to 15,000 micropores/µm², and
an average opening ratio of the anodic oxide film is equal to or higher than 51%.

2. The cell culture carrier according to Claim 1, wherein an average opening diameter of the plurality of micropores is from 40 nm to 100 nm.

3. The cell culture carrier according to Claim 1 or 2, wherein a thickness of the cell culture carrier is from 10 µm to 300 µm.

4. A cell culture vessel comprising:
at least one culture well having a culture chamber accommodating a cell culture medium; and
the cell culture carrier according to any one of Claims 1 to 3 that has a front surface to which cells adhere and which is positioned inside the culture chamber, and that is disposed such that the cell culture medium fills the cell culture carrier from the front surface to a rear surface of the cell culture carrier.

5. The cell culture vessel according to Claim 4, wherein the cell culture carrier is disposed such that the front surface and the rear surface thereof are positioned inside the culture chamber.

6. The cell culture vessel according to Claim 4, further comprising an accommodation portion that has an accommodation chamber accommodating the cell culture medium in an amount greater than that of the cell culture medium accommodated in the culture chamber,
wherein the cell culture carrier configures at least part of a bottom plate of the culture well,
the bottom plate of the culture well is disposed so as to be positioned inside the accommodation chamber, and
the culture chamber is in communication with the accommodation chamber through the plurality of micropores.

7. The cell culture vessel according to Claim 6,
wherein the culture well has an expansion portion that is in the form of a flat plate extending toward a side portion of the accommodation portion from a side edge of the bottom plate, and
the accommodation portion has a supporter that supports the expansion portion in the side portion thereof.

8. The cell culture vessel according to Claim 7,
wherein the expansion portion is formed of another anodic oxide film having a plurality of micropores penetrating the another anodic oxide film in a thickness direction and is integrally formed with the cell culture carrier.
